# NEUE EUROPÄISCHE PATENTSCHRIFT

(11) **EP 1 501 776 B2**
(45) Veröffentlichungstag und Bekanntmachung des Hinweises auf die Entscheidung über den Einspruch: **12.10.2016**
(45) Hinweis auf die Patenterteilung: 05.03.2008
(21) Anmeldenummer: 03747125.7
(22) Anmeldetag: 25.04.2003
(51) Int. Cl.: C07C 43/11, C07C 41/03, C11D 1/72

(54) **C10-ALKANOLALKOXYLATE UND IHRE VERWENDUNG**
C10-ALKANOLALKOXYLATES AND THE USE THEREOF
C10ALCANOLALCOXYLATES ET LEUR UTILISATION

(30) Priorität: 26.04.2002 DE 10218754; 18.09.2002 DE 10243360
(43) Veröffentlichungstag der Anmeldung: 02.02.2005
(73) Patentinhaber: BASF SE, 67056 Ludwigshafen am Rhein (DE)
(72) Erfinder: RULAND, Alfred, 69198 Schriesheim (DE); SCHOLTISSEK, Martin, 67157 Wachenheim (DE); OETTER, Günter, 67227 Frankenthal (DE); TAEGER, Klaus, 67251 Freinsheim (DE)
(86) Internationale Anmeldenummer: PCT/EP2003/004334
(87) Internationale Veröffentlichungsnummer: WO 2003/091191

(56) Entgegenhaltungen:
- WO-A-01/04183
- WO-A-94/11330
- WO-A-94/11331
- DE-A- 4 237 178
- US-A- 2 921 089
- SE 92 034 78 mit englischer Übersetzung

## Beschreibung

Die vorliegende Erfindung betrifft die Verwendung von C₁₀-Alkanolalkoxylaten, derartige C₁₀-Alkanolalkoxylate und Verfahren zu ihrer Herstellung.

Alkoxylate von aliphatischen Alkoholen werden in großem Umfang als Tenside und Emulgatoren eingesetzt. Die Benetzungs- und Emulgatoreigenschaften hängen dabei stark von der Art des Alkohols und der Art und Menge der Alkoxid-Addukte ab.

WO 94/11331 betrifft die Verwendung von Alkoxylaten von 2-Propylheptanol in Detergenzzusammensetzungen zur Entfettung harter Oberflächen. Die Alkoxylate weisen 2 bis 16 Alkylenoxid-Gruppen auf. Vorzugsweise liegt der überwiegende Teil der Alkylenoxid-Gruppen in Form von Ethylenoxid vor. Gemäß der Beispiele werden ausschließlich ethoxylierte Alkohole eingesetzt. Es ist ferner beschrieben, dass die Alkohole zunächst mit Ethylenoxid und sodann mit Propylenoxid umgesetzt werden können. Für derartige Alkoxylate sind jedoch keine Beispiele oder Eigenschaften angegeben. Es wird ausgeführt, dass die beschriebenen Alkoxylate eine gute Detergenz- und Benetzungswirkung zeigen, verbunden mit einem geringen Schäumen. Zudem wird angegeben, dass die Alkoxylate einen erwünschten Verdickungseffekt in Formulierungen haben.

WO 94/11330 betrifft Alkoxylate von 2-Propylheptanol und deren Verwendung. In den Alkoxylaten liegt 2-Propylheptanol, zunächst mit 1 bis 6 mol Propylenoxid und sodann mit 1 bis 10 mol Ethylenoxid umgesetzt, vor. Gemäß den Beispielen wird ein zunächst mit 4 mol Propylenoxid und sodann mit 6 mol Ethylenoxid umgesetztes 2-Propylheptanol eingesetzt. Es wird angegeben, dass die Alkylenoxidaddukte ein verbessertes Verhältnis von Schaumverhalten zu Detergenzwirkung zeigen. Ferner ist angegeben, dass die Alkoxylate ein gutes Benetzungsverhalten zeigen. Sie werden in Detergenzzusammensetzungen zur Reinigung von Textilmaterialien eingesetzt.

Die US 2,508,036 betrifft die Verwendung von 2-n-Propylheptanolethoxilaten, die 5 bis 15 mol Ethylenoxid enthalten, als Netzmittel in wässrigen Lösungen. Es ist beschrieben, dass die Produkte als Tenside in Waschmitteln eingesetzt werden können. Verfahren zur Alkoxylierung von 2-Propylheptanol sind prinzipiell aus dem Stand der Technik bekannt. In der WO 01/04183 wird beispielsweise ein Verfahren zur Ethoxylierung von hydroxyfunktionellen Starterverbindungen beschrieben, das in Gegenwart einer Doppelmetallcyanid-Verbindung als Katalysator durchgeführt wird.

Aufgabe der vorliegenden Erfindung ist die Bereitstellung von Alkanolalkoxylaten, die als Emulgator, Schaumregulierer und als Netzmittel für harte Oberflächen geeignet sind. Die Alkoxylate sollen insbesondere ein gutes Emulgierverhalten und einen geringen Kontaktwinkel auf harten Oberflächen bei der Anwendung zeigen. Ferner sollen sie die Grenzflächenspannung in flüssigen Systemen vermindern. Die Alkoxylate sollen allgemein ein vorteilhaftes Eigenschaftsspektrum bei der Verwendung als Emulgator, Schaumregulierer oder als Netzmittel zeigen. Des weiteren sollen die Produkte ein günstiges ökologisches Profil aufweisen, d.h. nicht aquatoxisch sein: Werte EC 50 für Alge, Daphnie oder Fisch grösser 10 mg/l; sowie leicht abbaubar nach OECD 301 A - F sein. Dazu soll der Restalkoholgehalt gegenüber den Ethoxilaten reduziert sein. Damit soll der für eine Vielzahl von Anwendungen als störend empfundene Geruch verursacht durch den Restalkoholgehalt vermieden werden.

Die Aufgabe wird erfindungsgemäß gelöst durch die Verwendung von Alkoxylaten der allgemeinen Formel (I)

C₅H₁₁CH(C₃H₇)CH₂O(A)ₙ(CH₂CH₂O)ₘH (I)

mit der Bedeutung
- A: Propylenoxy,
- n: Zahl im Bereich von 1,2 bis 1,8,
- m: Zahl im Bereich von 2 bis 20,
als Emulgator, Schaumregulierer und als Netzmittel für harte Oberflächen. Es wurde erfindungsgemäß gefunden, dass die vorstehenden Alkoxylate der allgemeinen Formel (I) hervorragende Emulgatoreigenschaften zeigen und als nicht oder wenig schäumende Netzmittel für harte Oberflächen eingesetzt werden können. Die Alkoxylate zeigen geringe Kontaktwinkel bei der Benetzung harter Oberflächen und erlauben die Einstellung geringer Grenzflächenspannungen in flüssigen Systemen.

Damit sind die Alkoxylate der allgemeinen Formel (I) besonders vorteilhaft einsetzbar in Tensidformulierungen zur Reinigung harter Oberflächen, in Feuchthaltemitteln, kosmetischen, pharmazeutischen und Pflanzenschutzformulierungen, Lacken, Beschichtungsmitteln, Klebstoffen, Lederentfettungsmitteln, Formulierungen für die Metallverarbeitung, Lebensmittelindustrie, Wasserbehandlung, Papierindustrie, Fermentation, Mineralverarbeitung und in Emulsionspolymerisationen. Auf die einzelnen Anwendungsgebiete wird nachfolgend noch näher eingegangen.

In der allgemeinen Formel (I) bedeutet n eine Zahl im Bereich von 1,2 bis 1,8, bevorzugt 1,3 bis 1,7, insbesondere 1,4 bis 1,6, speziell etwa 1,5. m ist vorzugsweise eine Zahl im Bereich von 3 bis 14, besonders bevorzugt 3 bis 10.

In den erfindungsgemäßen Alkoxylaten liegen an den Alkoholrest anschließend zunächst Propylenoxy-Einheiten und daran anschließend Ethylenoxy-Einheiten vor. n und m bezeichnen dabei einen mittleren Wert, der sich als Durchschnitt für die Alkoxylate ergibt. Daher können n und m auch von ganzzahligen Werten abweichen. Bei der Alkoxylierung von Alkanolen wird im Allgemeinen eine Verteilung des Alkoxylierungsgrades erhalten, die in gewissem Umfang durch Einsatz unterschiedlicher Alkoxylierungskatalysatoren eingestellt werden kann. In den erfindungsgemäß verwendeten Alkoxylaten wurde das Alkanol zunächst mit Propylenoxid und sodann mit Ethylenoxid umgesetzt.

Die Erfindung betrifft auch Alkoxylate der allgemeinen Formel (I)

C₅H₁₁CH(C₃H₇)CH₂O(A)ₙ(CH₂CH₂O)ₘH (I)

mit der Bedeutung
- A: Propylenoxy,
- n: Zahl im Bereich von 1,2 bis 1,8,
- m: Zahl im Bereich von 3 bis 14.

Dabei ist n vorzugsweise eine Zahl im Bereich von 1,3 bis 1,7, insbesondere von 1,4 bis 1,6. Speziell bevorzugt hat n einen Wert von etwa 1,5. m ist vorzugsweise eine Zahl im Bereich von 3 bis 12, besonders bevorzugt von 3 bis 10, speziell 5 bis 10.

In der allgemeinen Formel (I) kann der Rest C₅H₁₁ die Bedeutung n-C₅H₁₁, C₂H₅CH(CH₃)CH₂ oder CH₃CH(CH₃)CH₂CH₂ haben. Es können auch Gemische von zweien oder mehreren dieser Verbindungen vorliegen. Beispielsweise kann sich bei den Alkoxylaten um Gemische handeln, wobei
70 bis 99 Gew.%, vorzugsweise 85 bis 96 Gew.-% Alkoxylate A1 vorliegen, in denen C₅H₁₁ die Bedeutung n-C₅H₁₁ hat, und
1 bis 30 Gew.-%, vorzugsweise 4 bis 15 Gew.-% Alkoxylate A2, in denen C₅H₁₁ die Bedeutung C₂H₅CH(CH₃)CH₂ und/oder CH₃CH(CH₃)CH₂CH₂ hat.

Die allgemeine Formel (I) umfasst damit auch derartige Gemische. In der allgemeinen Formel (I) hat der Rest C₃H₇ vorzugsweise die Bedeutung n-C₃H₇.

Das in den Alkoxylaten vorliegende Propylheptanol kann ausgehend von Valeraldehyd durch Aldolkondensation und nachfolgende Hydrierung erhalten werden. Die Herstellung von Valeraldehyd und den entsprechenden Isomeren erfolgt durch Hydroformylierung von Buten, wie beispielsweise in US 4,287,370; Beilstein E IV 1, 32 68, Ullmanns Encyclopedia of Industrial Chemistry, 5. Auflage, Band A1, Seiten 323 und 328 f beschrieben. Die nachfolgende Aldolkondensation ist beispielsweise beschrieben in US 5,434,313 und Römpp, Chemie Lexikon, 9. Auflage, Stichwort "Aldol-Addition" Seite 91. Die Hydrierung des Aldolkondensationsproduktes folgt allgemeinen Hydrierbedingungen.

Des weiteren kann 2-Propylheptanol durch Kondensation von 1-Pentanol (als Mischung der entsprechenden Methylbutanole-1) in Gegenwart von KOH bei erhöhten Temperaturen hergestellt werden, siehe z.B. Marcel Guerbet, C.R. Acad Sei Paris 128, 511, 1002 (1899). Des weiteren ist auf Römpp, Chemie Lexikon, 9. Auflage, Georg Thieme Verlag Stuttgart, und die dort genannten Zitate sowie Tetrahedron, Vol. 23, Seiten 1723 bis 1733, hinzuweisen.

Die Alkoxylaten der allgemeinen Formel (I) können erfindungsgemäß erhalten werden durch Umsetzung von Alkoholen der allgemeinen Formel C₅H₁₁CH(C₃H₇)CH₂OH zuerst mit Propylenoxid und sodann mit Ethylenoxid unter Alkoxylierungsbedingungen. Geeignete Alkoxylierungsbedingungen sind beispielsweise in Nikolaus Schönfeldt, Grenzflächenaktive Äthylenoxid-Addukte, Wissenschaftliche Verlagsgesellschaft mbH Stuttgart, 1984 beschrieben. Die Alkoxylierung kann beispielsweise unter Verwendung von alkalischen Katalysatoren wie Alkalihydroxiden, Alkalialkoholaten durchgeführt werden. Durch den Einsatz dieser Katalysatoren resultieren spezielle Eigenschaften, insbesondere der Verteilung des Alkoxylierungsgrades.

Die Alkoxylierung kann zudem unter Verwendung von Lewis-saurer Katalyse mit den daraus resultierenden speziellen Eigenschaften durchgeführt werden, insbesondere in Gegenwart von BF₃ x H₃PO₄, BF₃ Dietherat, SbCl₅, SnCl₄ x 2 H₂O, Hydrotalcit. Geeignet als Katalysator sind auch Doppelmetallcyanid (DMC) Verbindungen.

Dabei kann der überschüssige Alkohol abdestilliert werden, oder das Alkoxylat kann durch einen Zwei-Stufen-Prozess gewonnen werden. Auch die Herstellung gemischter Alkoxylate aus beispielsweise EO und PO ist möglich, wobei sich an den Alkanolrest zunächst ein Polyethylenoxid-Block und dann ein Ethylenoxid-Block anschließen können, oder zunächst ein Ethylenoxid-Block und sodann ein Propylenoxid-Block. Bevorzugte Umsetzungsbedingungen sind nachstehend angegeben.

Vorzugsweise wird die Alkoxylierung durch starke Basen katalysiert, die zweckmäßigerweise in Form eines Alkalihydroxids oder Erdalkalihydroxids, in der Regel in einer Menge von 0,1 bis 1 Gew.-% bezogen auf die Menge des Alkanols zugesetzt werden, vergl. G.Gee et al., J. Chem. Soc. (1961), S. 1345; B. Wojtech, Makromol. Chem. 66, (1966), S. 180.

Auch eine saure Katalyse der Additionsreaktion ist möglich. Neben Bronstedsäuren eignen sich auf Lewissäuren wie z.B. AlCl₃ oder BF₃ Dietherat, BF₃ x H₃PO₄, SbCl₄ x 2 H₂O Hydrotalcit (Vergl. P.H. Plesch, The Chemistry of Cationic. Polymerization, Pergamon Press, New York (1963)). Geeignet als Katalysator sind auch Doppelmetallcyanid (DMC) Verbindungen.

Als DMC-Verbindung können prinzipiell alle dem Fachmann bekannten geeigneten Verbindungen verwendet werden.

Als Katalysator geeignete DMC-Verbindungen sind beispielsweise in der WO 99/16775 und der DE-A-10117273 beschrieben. Insbesondere sind für die Alkoxylierung Doppelmetallcyanid-Verbindung der allgemeinen Formel I als Katalysator geeignet:

M¹ₐ[M²(CN)_{b}(A)_{c}]_{d}·fM¹_{g}Xₙ·h(H₂O)·eL·kP (I),

in der
- M¹ mindestens ein Metallion, ausgewählt aus der Gruppe bestehend aus Zn²⁺, Fe²+, Fe³⁺, Co³⁺, Ni²⁺, Mⁿ²⁺, Co²⁺, Sn²+, Pb²⁺, Mo⁴⁺, Mo⁶⁺, Al³⁺, V⁴⁺, V⁵⁺, Sr²⁺, W⁴⁺, W⁶⁺, Cr²⁺, Cr3+, Cd²⁺, Hg²⁺, Pd²⁺, Pt²⁺, V²⁺, Mg²⁺, Ca²⁺, Ba²⁺, Cu²⁺, La³⁺, Ce³⁺, Ce⁴⁺, Eu³⁺, Ti³⁺, Ti⁴⁺, Ag⁺, Rh²⁺, Rh³⁺, Ru²⁺, Ru³⁺ ist,
- M² mindestens ein Metallion, ausgewählt aus der Gruppe bestehend aus Fe²⁺, Fe³⁺, Co²⁺, Co³⁺, Mn²⁺, Mn³⁺, V⁴⁺, V⁵⁺, Cr²⁺, Cr³⁺, Rh³⁺, Ru²⁺, Ir³⁺ ist,
- A und X unabhängig voneinander ein Anion, ausgewählt aus der Gruppe, bestehend aus Halogenid, Hydroxid, Sulfat, Carbonat, Cyanid, Thiocyanat, Isocyanat, Cyanat, Carboxylat, Oxalat, Nitrat, Nitrosyl, Hydrogensulfat, Phosphat, Dihydrogenphosphat, Hydrogenphosphat oder Hydrogencarbonat sind,
- L ein mit Wasser mischbarer Ligand ist, ausgewählt aus der Gruppe, bestehend aus Alkoholen, Aldehyden, Ketonen, Ethern, Polyethern, Estern, Polyestern, Polycarbonat, Harnstoffen, Amiden, primären, sekundären und tertiären Aminen, Liganden mit Pyridin-Stickstoff, Nitrilen, Sulfiden, Phosphiden, Phosphiten, Phosphanen, Phosphonaten und Phosphaten,
- k eine gebrochene oder ganze Zahl größer oder gleich Null ist, und
- P ein organischer Zusatzstoff ist,
- a, b, c, d, g und n so ausgewählt sind, dass die Elektroneutralität der Verbindung (I) gewährleistet ist, wobei c = 0 sein kann,
- e die Anzahl der Ligandenmoleküle eine gebrochenen oder ganze Zahl größer 0 oder 0 ist,
- f, h und m unabhängig voneinander eine gebrochene oder ganze Zahl größer 0 oder 0 sind.

Als organische Zusatzstoffe P sind zu nennen: Polyether, Polyester, Polycarbonate, Polyalkylenglykolsorbitanester, Polyakylenglykolglycidylether, Polyacrylamid, Poly(acrylamid-co-acrylsäure), Polyacrylsäure, Poly(acrylamid-co-maleinsäure), Polyacrylnitril, Polyalkylacrylate, Polyalkylinethacrylate, Polyvinylmethylether, Polyvinylethylether, Polyvinylacetat, Polyvinylalkohol, Poly-N-vinylpyrrolidon, Poly(N-vinylpyrrolidon-co-acrylsäure), Polyvinylmethylketon, Poly(4-vinylphenol), Poly(acrylsäure-co-styrol), Oxazolinpolymere, Polyalkylenimine, Maleinsäure- und Maleinsäureanhydridcopolymere, Hydroxyethylcellulose, Polyacetate, ionische oberflächen- und grenzflächenaktive Verbindungen, Gallensäure oder deren Salze, Ester oder Amide, Carbonsäureester mehrwertiger Alkohole und Glycoside.

Diese Katalysatoren können kristallin oder amorph sein. Für den Fall, dass k gleich null ist, sind kristalline Doppelmetallcyanid-Verbindungen bevorzugt. Im Fall, dass k größer null ist, sind sowohl kristalline, teilkristalline, als auch substantiell amorphe Katalysatoren bevorzugt.

Von den modifizierten Katalysatoren gibt es verschiedene bevorzugte Ausführungsformen. Eine bevorzugte Ausführungsform sind Katalysatoren der Formel (I), bei denen k größer null ist. Der bevorzugte Katalysator enthält dann mindestens eine Doppehnetallcyanid-Verbindung, mindestens einen organischen Liganden und mindestens einen organischen Zusatzstoff P.

Bei einer anderen bevorzugten Ausführungsform ist k gleich null, optional ist e auch gleich null und X ist ausschließlich ein Carboxylat, bevorzugt Formiat, Acetat und Propionat. Derartige Katalysatoren sind in der WO 99/16775 beschrieben. Bei dieser Ausführungsform sind kristalline Doppelmetallcyanid-Katalysatoren bevorzugt. Ferner bevorzugt sind Doppelmetallcyanid-Katalysatoren, wie in der WO 00/74845 beschrieben, die kristallin und plättchenförmig sind.

Die Herstellung der modifizierten Katalysatoren erfolgt durch Vereinigung einer Metallsalz-Lösung mit einer Cyanometallat-Lösung, die optional sowohl einen organischen Liganden L als auch einen organischen Zusatzstoff P enthalten können.

Anschließend werden der organische Ligand und optional der organische Zusatzstoff zugegeben. Bei einer bevorzugten Ausführungsform der Katalysatorherstellung wird zunächst eine inaktive Doppelmetallcyanid-Phase hergestellt und diese anschließend durch Umkristallisation in eine aktive Doppelmetallcyanidphase überfuhrt, wie in der PCT/EP01/01893 beschrieben.

Bei einer anderen bevorzugten Ausführungsform der Katalysatoren sind f, e und k ungleich Null. Dabei handelt es sich um Doppelmetallcyanid-Katalysatoren, die einen mit Wasser mischbaren organischen Ligand (im allgemeinen in Mengen von 0,5 bis 30 Gew.%) und einen organischen Zusatzstoff (im allgemeinen in Mengen von 5 bis 80 Gew.%) enthalten wie in der WO 98/06312 beschrieben. Die Katalysatoren können entweder unter starkem Rühren (24000U/Min mit Turrax) oder unter Rühren hergestellt werden wie in der US 5,158,922 beschrieben.

Insbesondere als Katalysator geeignet sind für die Alkoxylierung Doppelmetallcyanid-Verbindungen, die Zink, Kobalt oder Eisen oder zwei davon enthalten. Besonders geeignet ist beispielsweise Berliner Blau.

Bevorzugt werden kristalline DMC-Verbindungen eingesetzt. In einer bevorzugten Ausführungsform wird eine kristalline DMC-Verbindung vom Zn-Co-Typ als Katalysator verwendet, der als weitere Metallsalzkomponente Zinkacetat enthält. Derartige Verbindungen kristallisieren in monokliner Struktur und weisen einen plättchenförmigen Habitus auf. Derartige Verbindungen werden beispielsweise in der WO 00/74845 oder der PCT/EPO1/01893 beschrieben.

Als Katalysator geeignete DMC-Verbindungen können prinzipiell auf alle dem Fachmann bekannten Arten hergestellt werden. Beispielsweise können die DMC-Verbindungen durch direkte Fällung, "incipient wetness"-Methode, durch Herstellung einer Precursor-Phase und anschließende Umkristallisation hergestellt werden.

Die DMC-Verbindungen können als Pulver, Paste oder Suspension eingesetzt werden oder zu einem Formkörper verformt werden, in Formkörpern, Schäume oder ähnliches eingebracht werden oder auf Formkörper, Schäume oder ähnliches aufgebracht werden.

Die zur Alkoxylierung eingesetzte Katalysator-Konzentration, bezogen auf das Endmengengerüst, ist typischerweise kleiner als 2000 ppm, bevorzugt kleiner als 1000 ppm, insbesondere kleiner als 500 ppm, besonders bevorzugt kleiner als 100 ppm, beispielsweise kleiner als 50 ppm oder 35 ppm, insbesondere bevorzugt kleiner als 25 ppm.

Die Additionsreaktion wird bei Temperaturen von 90 bis 240°C, vorzugsweise von 120 bis 180°C, im geschlossenen Gefäß ausgeführt. Das Alkylenoxid oder die Mischung verschiedener Alkylenoxide wird der Mischung aus erfindungsgemäßem Alkanol(gemisch) und Alkali unter dem bei der gewählten Reaktionstemperatur herrschenden Dampfdruck des Alkylenoxidgemisches zugeführt. Gewünschtenfalls kann das Alkylenoxid mit bis zu etwa 30 bis 60 % mit einem Inertgas verdünnt werden. Dadurch wird eine zusätzliche Sicherheit gegen explosionsartige Polyaddition des Alkylenoxids gegeben.

Die Länge der Polyetherketten schwankt innerhalb des Reaktionsprodukts statistisch um einen Mittelwert, der im wesentlichen dem sich aus der Zusatzmenge ergebenden stöchiometrischen Wert entspricht.

In der Regel wird die Alkoxylierung in Gegenwart basischer Katalysatoren wie KOH in Substanz durchgeführt. Die Alkoxylierung kann jedoch auch unter Mitverwendung eines Lösungsmittels durchgeführt werden. Zur Herstellung der erfindungsgemäßen Alkoxylate werden die Alkohole zunächst mit einer geeigneten Menge an Propylenoxid und sodann mit einer geeigneten Menge an Ethylenoxid umgesetzt. Dabei wird eine Polymerisation des Alkylenoxids in Gang gesetzt, bei der es zwangsläufig zu einer statistischen Verteilung von Homologen kommt, deren Mittelwert vorliegend mit n und m angegeben wird.

Durch die erfindungsgemäß zunächst durchgeführte Propoxylierung und erst nachfolgende Ethoxylierung kann der Gehalt an Restalkohol in den Alkoxylaten vermindert werden, da Propylenoxid gleichmäßiger an die Alkoholkomponente addiert wird. Im Unterschied dazu reagiert Ethylenoxid vorzugsweise mit Ethoxylaten, so dass bei einer anfänglichen Verwendung von Ethylenoxid zur Umsetzung mit den Alkanolen sowohl eine breite Homologenverteilung als auch ein hoher Gehalt an Restalkohol resultieren. Die Vermeidung von größeren Mengen an im Produkt vorliegendem Restalkohol ist insbesondere aus Geruchsgründen vorteilhaft. Die erfindungsgemäß eingesetzten Alkohole haben in der Regel einen Eigengeruch, der durch die vollständige Alkoxylierung weitestgehend unterdrückt werden kann. Nach üblichen Verfahren erhaltene Alkoxylate weisen einen hohen Eigengeruch auf, der für viele Anwendungen störend ist.

Es ist erfindungsgemäß nicht notwendig und nicht erwünscht, dass ein großer Restgehalt an Alkohol in den erfindungsgemäßen Alkoxylaten vorliegt. Gemäß einer Ausführungsform der Erfindung sind die Alkoxylate und deren Gemische weitgehend frei von Alkoholen.

Die erfindungsgemäßen Alkoxylate zeigen eine verbesserte Netzung auf harten Oberflächen, insbesondere im Vergleich mit entsprechenden Alkoholen, die nur ethoxyliert wurden bzw. zunächst ethoxyliert und sodann propoxyliert wurden. Produkte, die zunächst ethoxyliert und sodann propoxyliert wurden, zeigen ein weitgehend gleiches Netzungsverhalten wie Produkte, die nur ethoxyliert wurden, aber die vorteilhaften erfindungsgemäßen Netzungseigenschaften werden nicht erhalten.

Das vorteilhafte Netzungsverhalten der erfindungsgemäßen Verbindungen kann beispielsweise durch Messungen des Kontaktwinkels auf Glas, Polyethylenoxid oder Stahl ermittelt werden. Aus dem verbesserten Netzungsverhalten folgt eine bessere Performance bei insbesondere schnellen Reinigungsprozessen. Dies ist insofern überraschend, da durch die Kettenverlängerung des Ausgangsalkohols üblicherweise die dynamischen und netzenden Eigenschaften vermindert werden. Mit den erfindungsgemäßen Alkoxylaten kann damit die Benetzungsgeschwindigkeit von wässrigen Formulierungen erhöht werden. Die erfindungsgemäßen Alkoxylate können damit auch als Solubilisatoren eingesetzt werden, die insbesondere das Netzvermögen von Netzhilfsmitteln auch in verdünnten Systemen nicht negativ, sondern positiv beeinflussen. Sie können zur Erhöhung der Löslichkeit von Netzhilfsmitteln in wässrigen Formulierungen eingesetzt werden, die nicht-ionische Tenside enthalten. Sie dienen insbesondere zur Erhöhung der Benetzungsgeschwindigkeit in wässrigen Netzmitteln.

Ferner dienen die erfindungsgemäßen Alkoxylate zur Verminderung der Grenzflächenspannung, beispielsweise in wässrigen Tensidformulierungen. Die verminderte Grenzflächenspannung kann beispielsweise durch die Pendant-Drop-Methode bestimmt werden. Hieraus ergibt sich auch eine bessere Wirkung der erfindungsgemäßen Alkoxylate als Emulgator oder Co-Emulgator. Die erfindungsgemäßen Alkoxylate können auch zur Verminderung der Grenzflächenspannung bei kurzen Zeiten von üblicherweise unter einer Sekunde bzw. zur Beschleunigung der Einstellung der Grenzflächenspannung in wässrigen Tensidformulierungen eingesetzt werden.

Die vorliegende Erfindung betrifft auch Reinigungs-, Netz-, Beschichtungs-, Klebe-, Lederentfettungs- oder Feuchthaltemittel oder kosmetische, pharmazeutische oder Pflanzenschutzformulierungen, die mindestens ein wie vorstehend definiertes Alkoxylat der allgemeinen Formel (I) enthalten. Die Mittel enthalten dabei vorzugsweise 0,1 bis 20 Gew.-% der Alkoxylate. Nachstehend werden bevorzugte Einsatzgebiete der erfindungsgemäßen Alkoxylate näher beschrieben.

Die erfindungsgemäßen Alkoxylate werden vorzugsweise in den folgenden Bereichen eingesetzt:
- Tensidformulierungen zur Reinigung harter Oberflächen: Geeignete Tensidformulierungen, die mit den erfindungsgemäßen Alkoxylaten additiviert werden können, sind beispielsweise in Formulating Detergents and Personal Care Products von Louis Ho Tan Tai, AOCS Press, 2000; beschrieben. Sie enthalten beispielsweise als weitere Komponenten Seife, anionische Tenside wie LAS oder Paraffinsulfonate oder FAS oder FAES, Säure wie Phosphorsäure, Amidosulfonsäure, Zitronensäure, Milchsäure, Essigsäure, andere organische und anorganische Säuren, Lösungsmittel wie Ethylenglykol, Isopropanol, Komplexbildner wie EDTA, NTA, MGDA, Phosphonate, Polymere wie Polyacrylate, Copolymere Maleinsäure-Acrylsäure, Alkalispender wie Hydroxide, Silicate, Carbonate, Parfümöle, Oxidationsmittel wie Perborate, Persäuren oder Trichloroisocyanursäure, Na oder K-dichloroisocyanurate, Enzyme; siehe auch Milton J. Rosen, Manilal Dahanayake, Industrial Utilization of Surfactants, AOCS Press, 2000 und Nikolaus Schönfeldt, Grenzflächenaktive Ethylenoxidaddukte. Hier sind auch Formulierungen für die anderen genannten Anwendungen im Prinzip abgehandelt. Es kann sich um Haushaltsreiniger wie Allzweckreiniger, Geschirrspülmittel für manuelles wie automatisches Geschirrspülen, Metallentfettung, Industrielle Applikationen wie Reinigungsmittel für die Nahrungsmittelindustrie oder Flaschenwäsche handeln. Es kann sich auch um Druckwalzen- und Druckplattenreinigungsmittel in der Druckindustrie handeln. Geeignete weitere Inhaltsstoffe sind dem Fachmann bekannt.
- Feuchthaltemittel, insbesondere für die Druckindustrie.
- Kosmetische, pharmazeutische und Pflanzenschutzformulierungen. Geeignete Pflanzenschutzformulierungen sind beispielsweise in der EP-A-0 050 228 beschrieben. Es können für Pflanzenschutzmittel übliche weitere Inhaltsstoffe vorliegen.
- Lacke, Beschichtungsmittel, Farben, Pigmentpräparationen sowie Klebstoffe in der Lack- und Folienindustrie.
- Lederentfettungsmittel.
- Faserverarbeitung und Hilfsmittel für die Papier- und Zellstoffindustrie.
- Metallverarbeitung wie Metallveredelung und Galvanobereich.
- Lebensmittelindustrie.
- Wasserbehandlung und Trinkwassergewinnung.
- Fermentation.
- Mineralverarbeitung und Staubkontrolle.
- Bauhilfsmittel.
- Emulsionspolymerisation und Herstellung von Dispersionen.
- Kühl- und Schmiermittel.

Solche Formulierungen enthalten üblicherweise Inhaltsstoffe wie Tenside, Gerüst-, Duft- und Farbstoffe, Komplexbildner, Polymere und andere Inhaltsstoffe. Typische Formulierungen sind beispielsweise in WO 01/32820 beschrieben. Weitere für unterschiedliche Anwendungen geeignete Inhaltsstoffe sind in EP-A-0 620 270, WO 95/27034, EP-A-0 681 865, EP-A-0 616 026, EP-A-0 616 028, DE-A-42 37 178 und US 5,340,495 beispielhaft beschrieben.

Allgemein können die erfindungsgemäßen Alkoxylate in allen Bereichen eingesetzt werden, in denen die Wirkung von grenzflächenaktiven Stoffen notwendig ist.

Die erfindungsgemäßen Strukturen weisen eine gegenüber bekannten Strukturen bessere Umwelt- und Hautverträglichkeit auf, so dass sie für eine Vielzahl von Anwendungsgebieten vorteilhaft geeignet sind.

Die Erfindung wird durch die nachstehenden Beispiele näher erläutert.

### BEISPIELE

### Herstellung der Alkoxylate

### Beispiel 1

### 2-Propylheptanol + 1,5 PO + 6 EO

790 g 2-Propylheptanol wurden mit 8,5 g KOH, 45 % in Wasser, in einem Autoklaven vorgelegt und zusammen bei 80°C und reduziertem Druck (ca. 20 mbar) entwässert. Dann wurden bei 120 bis 130°C 518 ml Propylenoxid zugegeben und bei dieser Temperatur unter erhöhtem Druck abreagieren lassen. Das Ende der Reaktion konnte an der Druckänderung beobachtet werden. Anschließend wurden 1470 ml Ethylenoxid bei 145 bis 155°C über längere Zeit bei erhöhtem Druck zudosiert und ebenfalls abreagieren lassen. Nach Spülen mit Inertgas und Abkühlen auf Raumtemperatur wurde der Katalysator durch Zugabe von 3,8 ml Eisessig neutralisiert.

### Beispiel 2

### 2-Propylheptanol + 1,5 PO + 8 EO

Umsetzung wie in Beispiel 1, die Alkoxylierung von 2-Propylheptanol erfolgte nach Zugabe von 45 %-iger KOH und anschließender Entwässerung bei ca. 80°C mit Propylenoxid und dann mit Ethylenoxid in den entsprechenden stöchiometrischen Verhältnissen unter Bedingungen wie in Beispiel 1 angegeben. Die Neutralisation wurde analog zu dem Beispiel 1 durchgeführt.

### Beispiel 3

### 2-Propylheptanol + 1,5 PO + 10 EO

630 g 2-Propytheptanol wurden mit 9,1 g KOH, 45 % in Wasser, in einem Autoklaven vorgelegt und zusammen bei 80°C und reduziertem Druck (ca. 20 mbar) entwässert. Dann wurden bei 120 bis 130°C 414 ml Propylenoxid zugegeben und bei dieser Temperatur unter erhöhtem Druck abreagieren lassen. Das Ende der Reaktion konnte an der Druckänderung beobachtet werden. Anschließend wurden 1990 ml Ethylenoxid bei 145 bis 155°C über längere Zeit bei erhöhtem Druck zudosiert und ebenfalls abreagieren lassen. Nach Spülen mit Inertgas und Abkühlen auf Raumtemperatur wurde der Katalysator durch Zugabe von 4,0 ml Eisessig neutralisiert.

### Vergleichsbeispiel V1

### 2-Propylheptanol + 8 EO

Umsetzung wie in Beispiel 1; auf die Umsetzung mit PO bei tieferer Temperatur wurde verzichtet, und 2-Propylheptanol wurde direkt bei 145 - 155°C mit 8 mol EO umgesetzt; die Neutralisation wurde analog zu Beispiel 1 durchgeführt.

### Vergleichsbeispiel V2

### 2-Propylheptanol + 8 EO + 1,5 PO

Umsetzung wie in Beispiel 1; aber 2-Propylheptanol wurde zuerst mit 8 mol EO bei 145 - 155°C umgesetzt und dann mit 1,5 mol PO bei 120 - 130°C umgesetzt; die Neutralisation wurde analog zu Beispiel 1 durchgeführt.

### Anwendungsbeispiele

Die erfindungsgemäßen Alkoxylate und die Vergleichsalkoxylate wurden zur Benetzung von Glas, Polyethylen und Stahl eingesetzt. Dabei wurde der Kontaktwinkel bei einer Konzentration von 0,2 g/l in Wasser bei einer Temperatur von 40°C gemessen. Die Ergebnisse sind in den nachstehenden Tabellen zusammengefasst.

### Grenzflächenspannung

Die Grenzflächenspannung wurde bei einer Konzentration von 1 g/l bei 25°C in Hexadecan und Olivenöl gemessen. Die Messung erfolgte nach der Pendant-Drop-Methode. Die Ergebnisse sind ebenfalls in den nachfolgenden Tabellen zusammengestellt.

### Kontaktwinkel auf V2A Stahl, [Grad]

| Zeit (sec) | 2-PH + 1,5 PO +8 EO | 2-PH + 8 EO + 1,5 PO | 2-PH + 8 EO | Demin. Wasser |
|---|---|---|---|---|
| 0,1 sec | 48 | 46 | 48 | 65 |
| 1 sec | 43 | 45 | 48 | 65 |
| 10 sec | 32 | 40 | 46 | 64 |

### Kontaktwinkel auf Polyethylen [Grad]

| Zeit (sec) | 2-PH + 1,5 PO + 8 EO | 2-PH + 8 EO + 1,5 PO | 2-PH + 8 EO | Demin. Wasser |
|---|---|---|---|---|
| 0,1 sec | 57 | 58 | 65 | 96 |
| 1 sec | 52 | 57 | 64 | 96 |
| 10 sec | 40 | 54 | 64 | 95 |

### Kontaktwinkel auf Glas [Grad]

| Zeit (sec) | 2-PH+1,5PO +8EO | 2-PH+8EO+ 1,5 PO | 2-PH+8EO | Demin. Wasser |
|---|---|---|---|---|
| 0,1 sec | 37 | 39 | 38 | 41 |
| 1sec | 32 | 33 | 32 | 40 |
| 10 sec | 20 | 24 | 25 | 39 |

### Netzung auf Baumwolle, EN 1/72,23, 0,1 g/l, 2 g/l Soda in dest. Wasser

| | 2-PH + 1,5 PO + 8 EO | 2-PH + 8 EO + 1,5 PO | 2-PH + 8 EO |
|---|---|---|---|
| Zeit (sec) | 10 | 17 | 15 |

### Restalkohol 2-PH, ermittels Gaschromatographisch mit internem Standard

| | 2-PH + 1,5 PO +8EO | 2-PH + 8 EO + 1,5 PO | 2-PH + 8 EO |
|---|---|---|---|
| g/100 g | 0,8 | 1,9 | 4,3 |

### Grenzflächenspannung, Pendant Drop Methode, 1 g/l, 25°C, Werte nach 10 min.

| (mN/m) | 2-PH + 1,5 PO + 8 EO | 2-PH + 8 EO + 1,5 PO | 2-PH + 8 EO |
|---|---|---|---|
| Hexadekan | 7,7 | 13,9 | 13,2 |
| Olivenöl | 5,2 | 8,0 | 8,4 |

### 2. Beispiel

### Kontaktwinkel auf V2A Stahl, [Grad]

| Zeit (sec) | 2-PH + 1,5 PO +6EO | 2-PH + 6 EO + 1,5 PO | 2-PH + 6 EO | Demin. Wasser |
|---|---|---|---|---|
| 0,1 sec | 42 | 46 | 48 | 65 |
| 1 sec | 35 | 43 | 46 | 65 |
| 10 sec | 23 | 37 | 42 | 64 |

### Kontaktwinkel auf Polyethylen [Grad]

| Zeit (sec) | 2-PH + 1,5 PO + 6 EO | 2-PH + 6 EO + 1,5 PO | 2-PH + 6 EO | Demin. Wasser |
|---|---|---|---|---|
| 0,1 sec | 55 | 56 | 62 | 96 |
| 1 sec | 48 | 54 | 61 | 96 |
| 10 sec | 36 | 48 | 59 | 95 |

### Kontaktwinkel auf Glas [Grad]

| Zeit (sec) | 2-PH + 1,5 PO + 6 EO | 2-PH + 6 EO + 1,5 PO | 2-PH + 6 EO | Demin. Wasser |
|---|---|---|---|---|
| 0,1 sec | 28 | 32 | 36 | 42 |
| 1 sec | 21 | 24 | 31 | 41 |
| 10 sec | 9 | 17 | 22 | 40 |

### Netzung auf Baumwolle, EN 1772, 23°C, 1 g/l, 2 g/l Soda in dest. Wasser

| | 2-PH+1,5PO +6EO | 2-PH + 6 EO + 1,5 PO | 2-PH+6EO |
|---|---|---|---|
| g/100 g | 1,4 | 13 | 10 |

### Restalkohol 2-PH, ermittelt gaschromatographisch mit internem Standard

| | 2-PH + 1,5 PO + 6 EO | 2-PH + 6 EO + 1,5 PO | 2-PH + 6 EO |
|---|---|---|---|
| g/100 g | 1,4 | 2,7 | 7,8 |

### Granzflächenspannung, Pendant Drop Methode, 1 g/l, 25°C, Werte nach 10 min

| (mN/m) | 2-PH + 1,5 PO +6EO | 2-PH + 6 EO + 1,5 PO | 2-PH + 6 EO |
|---|---|---|---|
| Hexadekan | 8,3 | 11,1 | 13,1 |
| Olivenöl | 6,7 | 7,5 | 9,0 |

Je kleiner der Kontaktwinkel und je kürzer die Zeit für seine Einstellung, desto besser ist die Netzung. Je kleiner die Grenzflächenspannung ist, desto größer sind die Grenzflächenaktivität und das Emulgiervermögen.

## Patentansprüche

1. Verwendung von Alkoxylaten der allgemeinen Formel (I)
C₅H₁₁CH(C₃H₇)CH₂O(A)ₙ(CH₂CH₂O)ₘH (I)
mit der Bedeutung
A Propylenoxy,
n Zahl im Bereich von 1,2 bis 1,8,
m Zahl im Bereich von 2 bis 20,
als Emulgator, Schaumregulierer und als Netzmittel für harte Oberflächen.

2. Verwendung nach Anspruch 1 in Tensidformulierungen zur Reinigung harter Oberflächen, in Feuchthaltemitteln, kosmetischen, pharmazeutischen und Pflanzenschutzformulierungen, Lacken, Beschichtungsmitteln, Klebstoffen, Lederentfettungsmitteln, Formulierungen für die Metallverarbeitung, Lebensmittelindustrie, Wasserbehandlung, Papierindustrie, Fermentation, Mineralverarbeitung und in Emulsionspolymerisationen.

3. Verwendung nach Anspruch 1 oder 2, **dadurch gekennzeichnet, dass** in der allgemeinen Formel (I) der Rest C₅H₁₁ die Bedeutung n-C₅H₁₁, C₂H₅CH(CH₃)CH₂ oder CH₃CH(CH₃)CH₂CH₂ hat oder Gemische von zweien oder mehreren dieser Verbindungen vorliegen.

4. Alkoxylat der allgemeinen Formel (I)
C₅H₁₁CH(C₃H₇)CH₂O(A)ₙ(CH₂CH₂O)ₘH (I)
mit der Bedeutung
A Propylenoxy,
n Zahl im Bereich von 1,2 bis 1,8,
m Zahl im Bereich von 3 bis 14.

5. Alkoxylat nach Anspruch 4, **dadurch gekennzeichnet, dass** in der allgemeinen Formel (I) n eine Zahl im Bereich von 1,3 bis 1,7 und m eine Zahl im Bereich von 3 bis 12 bedeuten.

6. Alkoxylat nach Anspruch 4 und 5, **dadurch gekennzeichnet, dass** in der allgemeinen Formel (I) der Rest C₅H₁₁ die Bedeutung n-C₅H₁₁, C₂H₅CH(CH₃)CH₂ oder CH₃CH(CH₃)CH₂CH₂ hat oder Gemische von zweien oder mehreren dieser Verbindungen vorliegen.

7. Verfahren zur Herstellung von Alkoxylaten gemäss einem der Ansprüche 4 bis 6 durch Umsetzung von Alkoholen der allgemeinen Formel C₅H₁₁CH(C₃H₇)CH₂OH zuerst mit Propylenoxid und sodann mit Ethylenoxid unter Alkoxylierungsbedingungen, wobei die Alkoxylierung in Gegenwart einer Doppelmetallcyanid-Verbindung als Katalysator erfolgen kann.

8. Verfahren nach Anspruch 7, **dadurch gekennzeichnet, dass** die Alkohole der allgemeinen Formel C₅H₁₁CH(C₃H₇)CH₂OH durch alkalische Dimerisierung von Valeraldehyd zu einem α,β-ungesättigten Aldehyd und nachfolgende Hydrierung erhalten werden.

9. Reinigungs-, Netz-, Beschichtungs-, Klebe-, Lederentfettungs- oder Feuchthaltemittel oder kosmetische, pharmazeutische oder Pflanzenschutzformulierung, enthaltend mindestens ein Alkoxylat der allgemeinen Formel (I), wie es in einem der Ansprüche 1 bis 6 definiert ist.

## Claims

1. The use of alkoxylates of the formula (I)
C₅H₁₁CH(C₃H₇)CH₂O(A)ₙ(CH₂CH₂O)ₘH (I)
where
A is propyleneoxy,
n is a number in the range from 1.2 to 1.8,
m is a number in the range from 2 to 20,
as emulsifier, foam regulator and as wetting agent for hard surfaces.

2. The use according to claim 1 in surfactant formulations for the cleaning of hard surfaces, in humectants, cosmetic, pharmaceutical and crop protection formulations, paints, coating compositions, adhesives, leather degreasing compositions, formulations for metalworking, food industry, water treatment, paper industry, fermentation, mineral processing and in emulsion polymerizations.

3. The use according to claim 1 or 2, wherein, in the formula (I), the radical C₅H₁₁ has the meaning n-C₅H₁₁, C₂H₅CH(CH₃)CH₂ or CH₃CH(CH₃)CH₂CH₂, or mixtures of two or more of these compounds are present.

4. An alkoxylate of the formula (I)
C₅H₁₁CH(C₃H₇)CH₂O(A)ₙ(CH₂CH₂O)ₘH (I)
where
A is propyleneoxy,
n is a number in the range from 1.2 to 1.8,
m is a number in the range from 3 to 14.

5. The alkoxylate according to claim 4, wherein, in the formula (I), n is a number in the range from 1.3 to 1.7 and m is a number in the range from 3 to 12.

6. The alkoxylate according to claims 4 and 5, wherein, in the formula (I), the radical C₅H₁₁ has the meaning n-C₅H₁₁, C₂H₅CH(CH₃)CH₂ or CH₃CH(CH₃)CH₂CH₂, or mixtures of two or more of these compounds are present.

7. A process for the preparation of alkoxylates according to any of claims 4 to 6 by reacting alcohols of the formula C₅H₁₁CH(C₃H₇)CH₂OH firstly with propylene oxide and then with ethylene oxide under alkoxylation conditions, wherein the alkoxylation can be carried out in the presence of a double-metal cyanide compound as catalyst.

8. The process according to claim 7, wherein the alcohols of the formula C₅H₁₁CH(C₃H₇)CH₂OH are obtained by alkaline dimerization of valeraldehyde to give an α, β-unsaturated aldehyde and subsequent hydrogenation.

9. A cleaning, wetting, coating, adhesive, leather degreasing composition or humectant or cosmetic, pharmaceutical or crop protection formulation comprising at least one alkoxylate of the formula (I) as defined in any of claims 1 to 6.

## Revendications

1. Utilisation d'alcoxylates de formule générale (I) :
C₅H₁₁CH(C₃H₇)CH₂O(A)ₙ(CH₂CH₂O)ₘH (I)
avec les significations suivantes :
A propylènoxy,
n un nombre compris dans l'intervalle de 1,2 à 1,8,
m un nombre compris dans l'intervalle de 2 à 20,
comme émulsionnant, régulateur de moussage et comme agent mouillant pour des surfaces dures.

2. Utilisation selon la revendication 1 dans des formulations tensioactives pour le nettoyage de surfaces dures, dans des produits humidifiants, des formulations cosmétiques, pharmaceutiques et de protection des plantes, des peintures, des agents de revêtement, des colles, des agents de dégraissage du cuir, des formulations pour le traitement des métaux, l'industrie alimentaire, le traitement de l'eau, l'industrie du papier, la fermentation, le traitement minéral et dans des polymérisations en émulsion.

3. Utilisation selon la revendication 1 ou 2, **caractérisée en ce que** dans la formule générale (I), le radical C₅H₁₁ a la signification n-C₅H₁₁, C₂H₅CH(CH₃)CH₂ ou CH₃CH(CH₃)CH₂CH₂ ou on a des mélanges de deux de ces composés, ou plus.

4. Alcoxylate de formule générale (I) :
C₅H₁₁CH(C₃H₇)CH₂O(A)ₙ(CH₂CH₂O)ₘH (I)
avec les significations suivantes :
A propylènoxy,
n un nombre compris dans l'intervalle de 1,2 à 1,8,
m est un nombre compris dans l'intervalle de 3 à 14.

5. Alcoxylate selon la revendication 4, **caractérisé en ce que** dans la formule générale (I), n représente un nombre compris dans l'intervalle de 1,3 à 1,7 et m est un nombre compris dans l'intervalle de 3 à 12.

6. Alcoxylate selon la revendication 4 et 5, **caractérisé en ce que** dans la formule générale (I), le radical C₅H₁₁ a la signification n-C₅H₁₁, C₂H₅CH(CH₃)CH₂ ou CH₃CH(CH₃)CH₂CH₂ ou on a des mélanges de deux de ces composés, ou plus.

7. Procédé de fabrication d'alcoxylates selon l'une des revendications 4 à 6, par réaction d'alcools de formule générale C₅H₁₁CH(C₃H₇)CH₂OH d'abord avec de l'oxyde de propylène et ensuite avec de l'oxyde d'éthylène dans des conditions d'alcoxylation, l'alcoxylation pouvant s'effectuer en présence d'un composé de cyanure de métal double comme catalyseur.

8. Procédé selon la revendication 7, **caractérisé en ce que** les alcools de formule générale C₅H₁₁CH(C₃H₇)CH₂OH sont obtenus par dimérisation alcaline de valéraldéhyde en un aldéhyde α,β-insaturé et hydrogénation consécutive.

9. Produit de nettoyage, agent mouillant, agent de revêtement, colle, agent de dégraissage du cuir ou produit humidifiant ou formulation cosmétique, pharmaceutique ou de protection des plantes, contenant au moins un alcoxylate de formule générale (I), tel que défini dans l'une des revendications 1 à 6.
